Europäisches Patentamt

European Patent Office

Office européen des brevets

(19)

(11) Veröffentlichungsnummer: **0 012 298**
**B1**

(12) **EUROPÄISCHE PATENTSCHRIFT**

(45) Veröffentlichungstag der Patentschrift: **19.05.82**

(21) Anmeldenummer: **79104810.1**

(22) Anmeldetag: **01.12.79**

(51) Int. Cl.³: **C 07 C 59/105,**
**C 07 D 307/32**

(54) Verfahren zur Reindarstellung von Kaliumribonat und Ribonolacton.

(30) Priorität: **06.12.78 DE 2852721**

(43) Veröffentlichungstag der Anmeldung:
**25.06.80 Patentblatt 80/13**

(45) Bekanntmachung des Hinweises auf die Patenterteilung:
**19.05.82 Patentblatt 82/20**

(84) Benannte Vertragsstaaten:
**CH DE FR GB**

(56) Entgegenhaltungen:
**DE - B - 1 148 991**
**US - A - 2 438 883**
**US - A - 3 709 912**

(73) Patentinhaber: **BASF Aktiengesellschaft**
**Carl-Bosch-Strasse 38**
**D-6700 Ludwigshafen (DE)**

(72) Erfinder: **Schmidt, Wolfram, Dr. Dipl.-Chem.**
**Im Rosengarten Ost 8**
**D-6701 Friedelsheim (DE)**
Erfinder: **Paust, Joachim, Dr. Dipl.-Chem.**
**Ringstrasse 3**
**D-6701 Neuhofen (DE)**

Courier Press, Leamington Spa, England.

Verfahren zur Reindarstellung von Kaliumribonat und Ribonolacton

Die vorliegende Erfindung betrifft ein Verfahren zur Reindarstellung von Kaliumribonat und Ribonolacton, ausgehend von Kaliumarabonat.

Ribonolacton ist bekanntlich ein wichtiges Zwischenprodukt für die Herstellung von Riboflavin (Vitamin $B_2$). Man erhält das Ribonolacton durch Lactonisierung von Ribonsäure oder von deren Salzen, den Ribonaten. Ribonate erhält man, indem man Glucose in wäßriger Natron- oder Kalilauge mit Sauerstoff zu einem Gemisch aus Na- bzw. K-Arabonat und Na- bzw. K-Formiat oxidiert, aus dieser Lösung das Arabonat durch Zugabe eines mit Wasser mischbaren Lösungsmittels isoliert und Arabonat anschließend durch Erhitzen in wäßriger Lösung auf 130 bis 140°C epimerisiert.

Da diese Epimerisierung stets nur bis zu einem Gleichgewicht von etwa 70% Arabonat mit 30% Ribonat führt, ist eine fraktionierte Kristallisation von Arabonat une Ribonat erforderlich. Hierzu eignen sich die Natrium- und Kaliumsalze jedoch nach bisherigen Kenntnissen nicht, da diese sehr wasserlöslich sind und sich kaum in ihrer Löslichkeit unterscheiden. Infolgedessen versetzt man im allgemeinen die Natrium- oder Kaliumarabonate und -ribonate enthaltenden wäßrigen Lösungen mit Calciumchlorid, worauf nach dem Abkühlen das Calciumarabonat bevorzugt auskristallisiert (vgl. Japan. Patentveröffentlichung 4525/1955). Eine Variante dieser Verfahrensweise besteht darin, das Na- bzw. K-Arabonat zunächst in das Ca-Arabonat zu überführen, dieses abzutrennen und die Epimerisierung anschließend vorzunehmen (vgl. US 2 438 882).

Nachteilig an den oben beschriebenen, bisher üblichen Verfahren ist,

1. daß die Natrium- bzw. Kaliumsalze zwecks Trennung der Arabonate und Ribonate erst in die Calciumsalze überführt werden müssen,

2. daß die Kristallisation der Calciumsalze sehr viel Zeit (mehrere Tage) in Anspruch nimmt und

3. daß man bei fraktionierten Kristallisation der genannten epimeren Calciumsalze nur ein Ribonat von nur etwa 80%iger Reinheit erhalten kann.

Zur Erzielung höherer Reinheiten überführte man die Ca-Salze in andere Salze, die durch weitere Kristallisation gereinigt werden können. Bekannt sind Trennungen über die Zink, Cadmium- oder Quecksilbersalze, die aber aufgrund der Giftigkeit der Metallsalze und der heirdurch bedingten Abwasserprobleme für ein technisches Verfahren ungeeignet sind.

Die ebenfalls für diesen Zweck verwendeten Eisensalze sind zwar nicht giftig, haben aber andere entscheidende Nachteile. So bedarf es beispielsweise zur Überführung der Calciumsalze in die Eisensalze eines 3- bis 5-stündigen Erhitzens auf 80 bis 100°C, was technisch unattraktiv ist. Außerdem gelingt die Überführung des Eisenribonats in Ribonolacton nur mit Ausbeuten von etwa 80 bis 85%, was für eine Technische Synthese unbefriedigend ist.

Es war daher Aufgabe der Erfindung, eine Möglichkeit zu finden, das für die Herstellung von Vitamin $B_2$ benötigte reine Ribonolacton unter Vermeidung der Nachteile der bekannten Verfahren auf technisch vorteilhafte Weise herzustellen. Hierzu war es notwendig, das Arabonat und Ribonat aus dem bei der Epimerisierung anfallenden Gemisch auf weniger aufwendige Weise zu trennen und außerdem ein vorteilhafteres Verfahren für die Feinreinigung von Ribonat bzw. Ribonolacton zu entwickeln.

Es wurde nun überraschenderweise gefunden, daß man das Kaliumribonat in einer wäßrigen Kaliumarabonat und Kaliumribonat enthaltenden Lösung entgegen allen Erwartungen auf recht einfache Weise stark anreichern kann, wenn man die wäßrige Lösung mit 40 bis 900 Gew.%, bezogen auf die Menge des Wassers, eines wasserlöslichen, nicht ionogenent oder praktisch nicht ionogenen, organischen Lösungsmittel versetzt und das anschließend hieran auskristallisierende Kaliumarabonat von der im wesentlichen das Kaliumribonat enthaltenden Lösung abtrennt.

Dieser Kristallisationsvorgang nimmt nur etwa 1 bis 5 Stunden in Anspruch, führt jedoch trotzdem zu einer Reinheit des Ribonats wie nach den bekannten Verfahren über die Calciumsalze. Die Möglichkeit einer so vorteilhaften Trennoperation ist deshalb überraschend, weil sich die Löslichkeiten von K-Arabonat und K-Ribonat in den wäßrig-organischen Lösungen nur etwa um den Faktor 2 unterschieden (zum Vergleich: die Löslichkeit der Ca-Salze in Wasser unterscheidet sich um den Faktor 80).

Es wurde weiterhin überraschenderweise gefunden, daß es möglich ist, aus einem Gemisch aus Kaliumribonat und Kaliumarabonat, in dem das Kaliumribonat zu 70 oder mehr Gew.% enthalten ist, durch fraktionierte Kristallisation aus Wasser bei niederen Temperaturen hochreines Kaliumribonat (95 bis 98%ige Reinheit) zu erhalten.

Es wurde weiterhin überraschenderweise gefunden, daß es möglich ist, aus einem Gemisch aus Ribonolacton und Arabolacton, in dem das Ribonolacton zu 70 oder mehr Gew.% enthalten ist, durch fraktionierte Kristallisation aus geeigneten Lösungsmitteln wie insbesondere Dioxan and Äthylenglykomonomethyläther hochreines Ribonolacton (95 bis 98 %iger Reinheit) zu erhalten.

Gegenstand der Erfindung ist somit ein Verfahren zur Gewinnung von Kaliumribonat oder Ribonolacton aus wässrigen, Kaliumribonat und Kaliumarabonat enthaltenden Lösungen, wie sie bei der Epimerisierung von Kaliumarabonat bei 130 bis 140°C in wässriger Lösung anfallen, das dadurch gekenzeichnet ist, daß man.

a) zur Gewinnung der Kaliumribonats

— diese wässrigen Lösungen mit 40 bis 900 Gew.%, bezogen auf die Menge des Wassers, eines wasserlöslichen nicht ionogenen oder Praktisch nicht ionogenen organischen Lösungsmittels versetzt,

— das anschließend hieran auskristallisierende Kaliumarabonat abtrennt,

— aus der verbleibenden, im wesentlichen Kaliumribonat neben wenig unumgesetzten Kaliumarabonat ethaltenden Lösung das Lösungsmittel und ggf. einen Teil des Wassers abdestilliert und

— das Kaliumribonat aus der nunmehr rein waßrigen konzentrierten Lösung bei niederen Temperaturen auskristallisieren läßt, bzw. daß man

b) zur Gewinnung von Ribonolacton entweder das gemäß a) erhaltene reine Kaliumribonat in an sich bekannter Weise lactonisiert

— oder aber das nach der gemäß a) erfolgten Abtrennung von auskristallisiertem Kaliumarabonat in der verbleibenden Lösung enthaltende Gemisch, das im wesentlichen Kaliumribonat neben wenig unumgesetzten Kaliumarabonat enthält, in an sich bekannter Weise lactonisiert

— und aus dem Lactongemisch das Ribonolacton durch Kristallisation isoliert.

Zur Durchführung des erfindungsgemäßen Verfahrens epimerisiert man eine wäßrige Kaliumarabonatlösung in an sich bekannter Weise durch Erhitzen auf 130 bis 140°C.

Die hierbei erhaltenen Lösungen enthalten im allgemeinen 5 bis 50, vorzugsweise 10 bis 30 Gew% der Kaliumsalze der beiden epimeren Aldonsäuren, wobei der Anteil an Arabonat etwa 60 bis 80%, der von Ribonat etwa 40 bis 20% beträgt.

Als definitionsgemäße wasserlösliche, nicht ionogene, organische Lösungsmittel eignen sich insbesondere $C_1$- bis $C_4$-Alkanole wie Methanol, Äthanol, Isopropanol und n-Butanol; cycloaliphatische Äther wie Dioxan sowie mit Methanol oder Äthanol partiell verätherte Alkandiole mit 2 bis 3 C-Atomen. Besonders gut geeignet sind Methanol sowie der Monomethylund Monoäthyläther des Äthylenglykols. Auch Mischungen der definitionsgemäß en Lösungsmittel können verwendet werden.

Geht man von relativ konzentrierten Kaliumarabonat-/-ribonatlösungen aus, also von solchen, die 20 bis 40 Gew.% dieser Salze enthalten, so verwendet man die Lösungsmittel zweckmäßigerweise in Mengen von 60 bis 250 Gew.% des Wassers (=100%). Bei dieser Verfahrensweise erzielt man eine relativ schnelle Kristallisation, die etwa 1 bis 2 Stunden in Anspruch nimmt. Man erhält ein Kristallisat aus 90 bis 98% Arabonat und 10 bis 2% Ribonat sowie eine Flüssigphase, deren Feststoffgehalt zu 30 bis 20% aus Arabonat und zu 70 bis 80% aus Ribonat besteht. Eine etwas bessere Trennung in eine überwiegend Arabonat enthaltende Kristallphase und eine überwiegend Ribonat enthaltende Flüssigphase erhält man, wenn man von verdünnteren Lösungen der epimeren Salze ausgeht, also von solchen, die 5 bis 15 Gew.% dieser Salze enthalten. In diesem Fall verwendet man die Lösungsmittel zweckmäßigerweise in Mengen von 200 bis 400 Gew.% des Wassers. Geht man von den verdünnteren wäßrigen Arabonat-/Ribonatlösungen aus, so nimmt man die Kristallisation zweckmäßigerweise bei Temperaturen von 0 bis 40°C vor. Im Fall der Verwendung der konzentrierten Lösungen kann man dagegen Temperaturen zwischen 40 und 70°C anwenden. Dies ist verfahrenstechnisch wegen der kleinen Volumina und der Möglichkeit der Kühlung durch die normalen, auf Raumtemperatur befindliche Umgebung besonders vorteilhaft.

Innerhalb der angegebenen Bereiche für die K-Arabonat-/Ribonat-Konzentrationen, für die Temperatur und für Art und Menge des Lösungsmittels lassen sich die für die jeweiligen Anforderungen wirtschaftlich optimalen Kristallisationsbedingungen aufgrund einiger Vorversuche leicht ermitteln, wobei es sich in allen Fällen empfiehlt, die Kristallisation des K-Arabonats mit einigen Impfkristallen zu initiieren.

Die nach der Abtrennung der Kaliumarabonats anfallende überwiegend Kaliumribonat enthaltende Flüssigphase wird nach destillativer Entfernung des zugesetzten Lösungmittels zur Reindarstellung von Kaliumribonat bzw. von Ribonolacton verwendet.

Zur Reindarstellung von Kaliumribonat engt man die nunmehr rein wäßrige Lösung soweit ein, daß eine etwa 40 bis 60 Gew.%ige Lösung vorliegt, kuhlt diese auf Temperaturen zwischen etwa —2 und +10°C, vorzugsweise etwa 0 bis 5°C ab und impft die Lösung mit einigen Kaliumribonatkristallen an. Überraschenderweise kristallisiert hierbei das Kaliumribonat in sehr guten Ausbeuten in 95 bis 98%iger Reinheit aus. Das so erhaltene Produkt ist aufgrund seiner hohen Reinheit ohne weitere Reinigung für die Synthese von Riboflavin sehr gut geeignet.

Zur reindarstellung von Ribonolacton kann man nun entweder das reine Kaliumribonat in an sich bekannter Weise lactonisieren oder aber die nach Abtrennen von Kaliumarabonat und zugesetztem Lösungsmittel erhaltene rein wäßrige, überwiegend Kaliumribonat enthaltende Lösung verwenden. Die Kaliumsalze dieser rein wäßrigen Lösung von Kaliumribonat und wenig Kaliumarabonat lassen sich in an sich bekannter Weise, beispielsweise durch Behandlung mit Ionenaustauschern oder Einengen unter Zusatz von Schwefelsäure und anschließendes Abfiltrieren des Ausgefallenen Kaliumsulfats in die Lactone überführen. Man erhält so ein Rohprodukt, das aus 70 bis 80% Ribonolacton und 20 bis 30% Arabolacton besteht. Bisher war nicht bekannt, daß man ein solches Produkt durch Kristallisation reinigen kann. Die Ursache hierfür liegt wohl darin, daß die beiden Lactone in den gängigen organischen Lösungsmitteln nur sehr geringe Löslichkeitsunterschiede aufweisen.

Es wurde gefunden, daß es einige Lösungsmittel gibt, aus denen man Ribonolacton aus diesem Rohprodukt in 95 bis 98%iger Reinheit kristallisieren kann. Eine besonders gute Kristallisation erfolgt aus Dioxan oder aus Äthylenglykolmonomethyläther.

Die Löslichkeitsunterschiede zwischen Ribono- und Arabonolacton sind auch bei diesen Lösungsmitteln sehr gering:

10 g Arabonolacton sind löslich in 75 ml Dioxan bei 25°C

10 g Ribonolacton sind löslich in 85 ml Dioxan bei 25°c

10 g Arabonolacton sind löslich in 22 ml Äthylenglykolmonomethyläther bei 25°C

10 g Ribonolacton sind löslich in 24 ml Äthylenglykolmonomethyläther bei 25°C

Daher ist est überraschend, daß dennoch eine selektive Kristallisation von Ribonolacton möglich ist, wenn im Rohprodukt noch etwa 20 bis 30% Arabonolacton enthalten ist. Die Kristallisation aus Dioxan gelingt aus 30 bis 60%iger, vorzugsweise 40 bis 50%iger Lösung. Die Kristallisationstemperatur wählt man zwischen 0 und 50°C, vorzugsweise zwischen 5 und 30°C. In Methylglykol kristallisiert man aus 30 bis 60%iger Lösung, vorzugsweise bei 50% und bei Temperaturen zwischen 0 und 40°C, vorzugsweise im Bereich 0 bis 25°C. Allgemein empfiehlt es sich, die Kristallisierlösung mit wenigen Kristallen von Ribonolacton anzuimpfen. Auf diese Weise läßt sich hochreines Ribonolacton erhalten.

Das in der hierbei anfallenden Mutterlauge enthaltene Gemisch an Lacton (etwa 50% Arabolacton und 50% Ribonolacton) läßt sich mit KOH leicht wieder in ein Gemisch der Kaliumsalze überführen und erneut der erfindungsgemäßen Trennung zuführen.

Mit Hilfe des erfindungsgemäßen Verfahrens war es möglich, das für die Herstellung von Vitamin $B_2$ benötigte Ribonolacton auf technisch sehr vorteilhafte Weise herzustellen.

### Biespiel 1

250 g einer wäßrigen Lösung, die 30% Kaliumarabonat und 10% Kaliumribonat enthielt, wurde bei 90°C mit (350 ml) Äthylenglykolmonomethyläther versetzt und nach Zugabe einiger Impfkristalle an K-Arabonat zwei Stunden bei 60°C gehalten. Hierbei kristallisierten 71 g (entsprechend 71% des zur Epimerisierung eingesetzten K-Arabonats (aus. Das restliche K-Arabonat verblieb zusammen mit 25 g K-Ribonat in der Lösung, was bedeutet, daß das K-Ribonat in etwa 85%iger Reinheit anfiel.

Die Konzentration an K-Arabonat und K-Ribonat wurden chromatographisch an einem Ionenaustauscher bestimmt.

Die eingesetzte Lösung entstammte der Epimerisierung von 100 g K-Arabonat in 500 g Wasser bei 140°C. Diese Lösung wurde mit Aktivkohle entfärbt und vor der Kristallisation auf 250 = 200 ml (entsprechend den oben angegebenen Gewichtsverhältnissen) eingeengt.

### Beispiel 2

460 g (= 420 ml) einer wäßrigen Lösung, die 15% K-Arabonat und 6,5% K-Ribonat enthielt, wurde mit 840 ml Äthylenglykolmonomethyläther und einigen Kristallen K-Arabonat versetzt. Innerhalb einer Stunde kristallisierten hierause bei 25°C 69 g K-Arabonat (entsprechend 69% des zur Epimerisierung eingesetzten K-Arabonats) aus. 2 g K-Arabonat blieben zusammen mit 29 g K-Ribonat in der Lösung, was bedeutet, daß das K-Ribonat mit etwa 95%iger Reinheit anfiel. Wie in Beispiel 1 wurde die Konzentration der epimeren Verbindung chromatographisch bestimmt.

Die eingesetzte Lösung entstammte einer wie in Beispiel 1 beschriebenen Epimerisierung von 100 g K-Arabonat, nur daß die dabei erhaltene Lösung nicht aur 200 ml sondern nur auf 360 ml eingeengt wurde.

### Beispiel 3

370 g (= 330 ml) einer wäßrigen Lösung, die 19% K-Arabonat und 8% K-Ribonat enthielt, wurde mit 630 ml Methanol und einigen Kristallen K-Arabonat versetzt. Innerhalb von 1 Std. kristallisierten bei 40°C hieraus 63 g K-Arabonat aus und 29 g K-Ribonat blieben, zusammen mit 6 g K-Arabonat in der Flüssigphase zurück.

Die Konzentration der epimeren Verbindungen wurde wie in Biespiel 1 chromatographischen bestimmt. Die eingesetzte Lösung entstammte einer wie in Beispiel 1 beschriebenen Epimerisierung von 100 g K-Arabonat, nur daß die dabei erhaltene Lösung nicht auf 200 ml sondern nur auf 330 ml eingeengt wurde.

### Beispiel 4

100 g Kaliumarabonat wurden in 320 ml Wasser gelöst und durch erhitzen auf 140°C epimerisiert. Die erhaltene Lösung wurde mit aktivkohle entfärbt und dann in der Wärme mit 480 ml Dioxan versetzt. Anschließend wurde auf Raumtemperatur abgekühlt. Es kristallisierten 65 g Kaliumarabonat aus. In der Mutterlauge verblieben 24 g Kaliumribonat neben 8 g Kaliumarabonat.

### Beispiel 5

Eine Lösung, enthaltend etwa 20 g Kaliumarabonat und 80 g Kaliumribonat, wie sie bei der Epimerisierung von 350 g Kaliumarabonat und anschließendem Ausfällen und Abtrennen der Hauptmenge des unumgesetzten Kaliumarabonats durch Zugabe von Äthylenglykolmonomethyläther erhalten wurde, wurde destillativ von dem Lösungsmittel befreit und auf etwa 200 ml eingeengt. Die so erhaltene etwa 50 %ige wäßrige Lösung wurde mit Eiswasser gekühlt und mit einigen Kristallen Kaliumribonat versetzt. In s Stunden kristallisierten etwa 60 g Kaliumribonat von 95 bis 98 %iger Reinheit aus. Die Ausbeute an reinem Kaliumribonat beträgt etwa 75 % des in der Mutterlauge enthaltenen Kaliumribonats.

### Beispiel 6

100 g eines Rohproduktes, bestehend aus etwa 80 % Ribonolacton und 20 % Arabonolacton, wie es bei der Epimerisierung von Kaliumarabonat, Ausfällen und Abtrennen der Hauptmenge des unumgesetzten Kaliumarabonats und Lactonisieren der in der Mutterlauge verbliebenen epimeren Kaliumsalze erhalten wurde, wurde in 150 ml Dioxan gelöst, die Lösung auf etwa 10°C abgekühlt und mit einigen Kristallen Ribonolacton angeimpft. Aus der Lösung kristallisierten 49 g eines 98 %reinen Ribonolactons aus. Die Ausbeute betrug 61 %, bezogen auf im Rohprodukt enthaltenem Ribonolacton.

### Beispiel 7

100 g eines Rohproduktes, bestehend aus 70% Ribonolacton und 30% Arabonolacton wurden in der Wärme in 100 ml Äthylenglykolmonomethyläther gelöst, die Lösung auf etwa 0°C gekühlt und mit einigen Ribonolactonkristallen geimpft. Aus der Lösung kristallisierten bei etwa 0°C etwa 45 g eines 95 % reinen Ribonolactons aus. Die Ausbeute betrug 64 %.

### Beispiel 8

100 g Kaliumarabonat wurden in 300 ml Wasser gelöst un wie üblich durch 4stündiges Erhitzen auf 130 bis 140°C epimerisiert. Die erhaltene Lösung wurde mit Aktivkohle entfärbt und in der Wärme mit 650 ml Methanol versetzt. Bei etwa 40°C kristallisierten 67 g unumgesetztes Kaliumarabonat aus, die erneut in die Epimerisierung eingesetzt werden können.

Aus der Mutterlauge wurde das Methanol abdestilliert. Die erhaltene rein wäßrige Lösung wurde in üblicher Weise über einen Kationenaustauscher in H+-Form laufen gelassen. Anschließend wurde das Wasser abdestilliert. Man erhielt 21 g eines rohen Ribonolactons, das noch etwa 20% Arabonolacton enthielt. Das erhaltene Rohprodukt wurde bei etwa 35°C mit wenigen Kristallen Ribonolacton angeimpft und auf Temperaturen zwischen 5 und 10°C abgekühlt. Man erhielt 10,4 g reines Ribonolacton.

### Beispiel 9

100 g Kaliumarabonat wurden in 300 ml Wasser gelöst und wie üblich durch 4stündiges Erhitzen auf 130 bis 140°C epimerisiert. Die erhaltene Lösung wurde mit Aktivkohle entfärbt und in der Wärme mit 650 ml Methanol versetzt. Bei etwa 40°C kristallisierten 67 g unumgesetztes Kaliumarabonat aus, die erneut in die Epimerisierung eingesetzt werden können. Aus der erhaltenen Mutterlauge wurde das Methanol abdestilliert und die erhaltene wäßrige Lösung auf 60 g eingeengt. Beim Abkühlen impft man mit wenigen Kristallen von Kaliumribonat an. Innerhalb von etwa 2 Stunden kristallisierten bei 5°C 17,4 g reines Kaliumribonat aus.

### Patentansprüche

1. Verfahren zur Gewinnung von Kaliumribonat oder Ribonolacton aus wässrigen, Kaliumribonat und Kaliumarabonat enthaltenden Lösungen, wie sie bei der Epimerisierung von Kaliumarabonat bei 130 bis 140°C in wässriger Lösung anfallen, dadurch gekenzeichnet, daß man

a) zur Gewinnung des Kaliumribonats

— diese wässrigen Lösungen mit 40 bis 900 Gew.%, bezogen auf die Menge des Wassers, eines wasserlöslichen nicht ionogenen oderpraktisch nicht ionogenen organischen Lösungsmittels versetzt,

— das anschließend hieran auskristallisierende Kaliumarabonat abtrennt,

— aus der verbleibenden, im wesentlichen Kaliumribonat neben wenig unumgesetzten Kaliumarabonat enthaltenden Lösung das Lösungsmittel und ggf. einen Teil des Wassers abdestilliert und

— das Kaliumribonat aus der nunmehr rein wäßrigen konzentrierten Lösung bei niederen Temperaturen auskristallisieren läßt, bzw. daß man

b) zur Gewinnung von Ribonolacton

— entweder das gemäß a) erhaltene reine Kaliumribonat in an sich bekannter Weise lactonisiert

— oder aber das nach der gemäß a) erfolgten Abtrennung von auskristallisierten Kaliumarabonat in der verbleibenden Lösung enthaltende Gemisch, das im wesentlichen Kaliumribonat neben wenig unumgesetztem Kaliumarabonat enthält, in an sich bekannter Weise lactonisiert und

— aus dem Lactongemisch das Ribonolacton duch Kristallisation isoliert.

2. Verfahren zur Anreicherung von Kaliumribonat in einer wäßrigen Kaliumarabonat und Kaliumribonat enthaltenden Lösung, dadurch gekennzeichnet, daß man die wäßrige Lösung mit 40 bis 900 Gew.%, bezogen auf die Menge des Wassers, eines wasserlöslichen nicht ionogenen oder Praktisch nicht ionogenen Lösungsmittel versetzt und das anschließend hieran auskristallisierende Kaliumarabonat von der im wesentlichen Kaliumribonat enthaltenden Lösung abtrennt.

3. Verfahren zur Gewinnung von Kaliumribonat aus einem Gemisch aus Kaliumribonat und Kaliumarabonat in dem das Kaliumribonat zu 70 oder mehr Gew.-% enthalten ist, dadurch gekennzeichnet, daß man das Gemisch aus konzentrierter wäßriger Lösung bei niederen Temperaturen Fraktioniert auskristallisieren läßt.

4. Verfahren zur Gewinnung von Ribonolacton aus einem Gemisch aus Ribonolacton und Arabonolacton, in dem das Ribonolacton zu 70 oder mehr Gew-% enthalten ist, dadurch gekennzeichnet, daß man das Gemisch aus konzentrierten Lösungen in Dioxan oder Äthylenglykolmonoethyläther bei niederen Temperaturen fraktioniert auskristallisieren läßt.

**Revendications**

1. Procéde pour l'obtention de ribonate de potassium ou de lactone ribonique à partir de solutions aqueuses contenant du ribonate de potassium et de l'arabonate de potassium, telles qu'elles résultent de l'épimérisation d'arabonate de potassium à une température de 130 à 140°C en solution aqueuse, caractérisé en ce que

a) pour l'obtention du ribonate de potassium,

— on additionne ces solutions aqueuses de 40 à 900% en poids, par rapport à la quantité d'eau, d'un solvant organique soluble dans l'eau, non ionogène au pratiquement non ionogène,

— on sépare l'arabonate de potassium qui se sépare par cristallisation à la suite de cette addition,

— on élimine par distillation, de la solution résiduelle qui contient, outre un peu de'arabonate de potassium n'ayant pas réagi, essentiellement du ribonate de potassium, le solvant et, le cas échéant, une partie de l'eau, et

— on provoque à basse température le séparation à l'état cristallin du ribonate de potassium, à partir de la solution aqueuse, désormais pure; et que

b) pour l'obtention de lactone ribonique,

— ou bien on lactonise, de façon connue en soi, le ribonate de potassium pur, obtenu suivant a),

— ou bien on lactonise, de façon connue en soi, le mélange qui est contenu dans la solution résiduelle à la suite de la séparation de l'arabonate de potassium cristallisé, effectuée suivant a), et qui contient essentiellement, outre un peu d'arabonate de potassium n'ayant pas réagi, du ribonate de potassium,

— et on isole par cristallisation la lactone ribonique à partir du mélange de lactones.

2. Procédé pour l'enrichissement de ribonate de potassium dans une solution aqueuse contenant de l'arabonate de potassium et du ribonate de potassium, caractérisé en ce qu'on additionne la solution aqueuse de 40 à 900% en poids, par rapport à la quantité d'eau, d'un solvant soluble dans l'eau, non ionogène ou pratiquement non ionogène, et on sépare, de la solution contenant essentiellement du ribonate de potassium, l'arabonate de potassium qui s'est séparé à l'état cristallin à la suite de cette addition.

3. Procédé pour l'obtention de ribonate de potassium à partir d'un mélange de ribonate de potassium et d'arabonate de potassium dans lequel le ribonate de potassium est contenu à raison de 70% en poids ou davantage, caractérisé en ce qu'on provoque la cristallisation fractionnée du mélange à partir d'une solution aqueuse concentrée à basse température.

4. Procédé pour l'obtention de lactone ribonique à partir d'un mélange de lactone ribonique et de lactone arabonique, dans lequel la lactone ribonique est contenue à raison de 70% en poids ou davantage, caractérisé en ce qu'on provoque la cristallisation fractionnée du mélange á partir de solutions concentrées dans le dioxanne ou l'éther monométhylique d'éthylène-glycol à basse température.

**Claims**

1. A process for the recovery of potassium ribonate and ribonolactone, from aqueous, potassium ribonate- and potassium arabonate-containing solutions which are formed in the epimerization of potassium arabonate at 130 to 140°C in aqueous solution, characterized in that

a) to obtain potassium ribonate, said aqueous solutions are mixed with from 40 to 900% by weight, based on the amount of water, of a water-soluble, non-ionic or virtually non-ionic, organic solvent, the potassium arabonate which hereupon crystallizes out is then separated off, the solvent, with or without part of the water, is distilled from the remaining solution which essentially contains potassium ribonate in addition to a small amount of unconverted potassium arabonate, and the potassium ribonate is allowed to crystallize out from the purely aqueous concentrated solution at a low temperature and

b) to obtain ribonolactone, either the pure potassium ribonate obtained according to a) is lactonized in the conventional manner, or the mixture contained in the solution which remains after separating of the crystallized-out potassium arabonate according to a), which mixture essentially contains potassium ribonate in addition to a small amount of unconverted potassium arabonate, is lactonized in the conventional manner, and the ribonolactone is isolated from the lactone mixture by crystallization.

2. A process for increasing the proportion of potassium ribonate in an aqueous solution containing potassium arabonate and potassium ribonate, characterized in that the aqueous solution is mixed with from 40 to 900% by weight, based on the amount of water, of a water-soluble non-ionic or virtually nonionic solvent, and the potassium arabonate which hereupon crystallizes out is separated from the solution which essentially contains potassium ribonate.

3. A process for the recovery of potassium ribonate from a mixture of potassium ribonate and potassium arabonate which contains 70%

**0 012 298**

by weight or more of potassium ribonate, characterized in that the mixture is subjected to fractional crystallization from concentrated aqueous solution at a low temperature.

4. A process for the recovery of ribonolactone from a mixture of ribonolactone and ara-bonolactone, which contains 70% by weight or more of the ribonolactone, characterized in that the mixture is subjected to fractional crystallisation from a concentrated solution in dioxane or ethylene glycol monoethyl ether at a low temperature.